# EUROPEAN PATENT APPLICATION

(11) **EP 4 663 008 A1**
(43) Date of publication of application: **17.12.2025**
(21) Application number: 24181609.9
(22) Date of filing: 12.06.2024
(51) Int. Cl.: A01H 4/00, A01G 22/00

(54) **A CASING FOR THE TRANSPLANTATION OF A PLANT**

(71) Applicant: Zostera-Marina ApS, 5000 Odense C (DK)
(72) Inventor: THOMSEN, Morten, 5000 Odense C (DK)
(74) Representative: Plougmann Vingtoft a/s

(57) **Abstract**

A casing assembly for a root of a plant and/or a seed of a plant configured to protect at least a part of the plant during transplantation of the plant, where the casing assembly comprises: at least one casing defining a casing volume for holding a part of a plant, where the casing comprises a first end part, a second end part, a first peripheral part and a second peripheral part, where the casing has a longitudinal axis extending from the first end part to the second end part, and a transverse axis extending from the first peripheral part to the second peripheral part, wherein the casing comprises a first side wall and a second side wall having a first inner surface and a second inner surface, respectively, defining the casing volume and a first outer surface and a second outer surface, respectively, wherein the casing volume has an opening, where the opening is positioned in the first end part and/or the first peripheral part and/or in a side wall of the casing, where the second end part comprises a force application part, so that when the casing is transplanted into a soil material the casing is pulled into the soil via the second end.

## Description

### Technical Field

A casing assembly for a root of a plant and/or a seed of a plant configured to protect at least a part of the plant during transplantation of the plant.

### Description

Climate change is an issue that awakens a lot of interest from different types of industries, where there has been a lot of focus on finding ways to reduce the carbon dioxide (CO2) in the atmosphere. There are numerous disclosures where industries utilize chemical or biological processes to attempt to capture CO2 from the atmosphere.

Biological solutions to increase carbon capture are widely used, where the transplantation of trees and other types of CO2 capturing plants have been practiced for centuries, as the plants are capable of capturing CO2 from the atmosphere for their growth.

However, there have also been numerous attempts to utilize plants to increase the carbon uptake in marine environments, where marine plants have capabilities to capture CO2 from both freshwater and seawater, and where marine plants may also have positive effects on marine life in areas where marine plants grow.

There have been multiple ways of attempting to increase the occurrence of marine plants in soil situated at the bottom of a body of water, such as a lake, ocean, river, etc, by transplanting marine plants in the soil or the sediments at the bottom of the waterbed. It has been shown that by increasing the transplantation of seaweed in soils that are in bodies of water, this results in increased uptake of CO2 and improved aquatic environment.

Furthermore, by transplanting water based plants into marine environments it is possible to increase biodiversity in the marine environment, as well as utilize marine based plants for protein production as well as utilizing the marine based plants for food production.

The marine environment is generally under pressure due to various factors such as elevated nutrients and oxygen depletion. Research shows that eelgrass has a very positive impact on the marine environment. Eelgrass has the ability to absorb and bind relatively large amounts of nutrients and provides further derived environmental services such as dampening of wave strokes, CO2 capture, oxygenation of the water, increasing light/visibility, hiding place for fry and generally increased biodiversity.

In order to benefit from the eelgrass's environmental services, eelgrass must exist locally and in large quantities. Large-scale transplantation of eelgrass is very resource-intensive and requires manual work with divers. The market is looking for new innovative solutions and methods that enable efficient large-scale transplantation, that is also beneficial for the plant's self-protection and vegetative growth

The transplantation of water-based plants, such as seaweed, or more specifically eelgrass, is often seen as problematic and the transplantation process is very time consuming. Seaweed has often a positive buoyancy in the body of water, which means that transplanted plants must be anchored in some way to the soil or the sediment to ensure that the plant succeeds in growing its roots into the soil or the sediment for it to be able to anchor itself in the bottom of the body of water. One way of transplanting seaweed has been to tie a plant to a weight, such as a metal nail, where the plant and nail are pushed into the soil from above, and where the nail reduces the buoyancy of the plant and may thereby hold the plant within the soil. However, there are numerous problems with this type of transplantation, as the introduction of a metal nail may not be healthy for the marine environment. Furthermore, by pushing the metal nail downwards from above, there is a risk that the exposed roots of the plant may become damaged during the introduction of the plant into the soil.

Thus, there is a need to improve the transplantation techniques, in which it may be possible to reduce the labour needed to transplant marine plants such as seaweed, and where the roots of the plant may be protected during transplantation.

In accordance with the invention there is provided a casing assembly for a root of a plant and/or a seed of a plant configured to protect at least a part of the plant during transplantation of the plant, where the casing assembly comprises: at least one casing defining a casing volume for holding a part of a plant, the casing comprises a first end part, a second end part, a first peripheral part and a second peripheral part, where the casing has a longitudinal axis extending from the first end part to the second end part, and a transverse axis extending from the first peripheral part to the second peripheral part, wherein the casing comprises a first side wall and a second side wall having a first inner surface and a second inner surface, respectively, defining the casing volume and a first outer surface and a second outer surface, respectively, wherein the casing volume has an opening, the opening is positioned in the first end part and/or the first peripheral part and/or in a side wall of the casing, and the second end part comprises a force application part, so that when the casing is transplanted into soil material the casing is pulled into the soil via the second end.

The casing presented in the present disclosure may be utilized to transplant plants, seedlings, seeds, germinated seeds and/or other types of plant substances in order to cultivate areas with multiple plants. Thus, in the following the reference to a plant may mean any of the above.

The casing may be configured to hold one or more plants within the casing volume, wherein the casing volume may be divided into one or more smaller volumes in order to separate the plants from each other while using one casing. The structure of the casing having a first end, a second end, a first transverse end and a second transverse end may be seen as a structure of the casing when the casing is introduced into the soil. Thus, the casing may have intermediate stages e.g. during preparations of the casing prior to transplantation. As an example, the reference to the second end and the force application part may be viewed in the moments prior to introduction of the casing in the soil.

The present casing assembly is intended to be utilized to protect the plant during transplantation and to provide an anchor to the plant after transplantation. The side wall of the casing is intended to enclose at least part of the plant, so that when a force is applied to the casing to introduce the casing into the soil, the casing may absorb a part of the force in order to protect the plant. This may be especially important when transplanting seedlings, as the roots of the seedlings may be delicate, and by providing the roots within the casing volume the forces used to transplant the seedling may be diverted away from the roots in order to ensure that the roots are intact when the plant has been introduced into the soil.

In many situations plants are transplanted from above, where the plant is pushed downwards into the soil, which may cause damage to the top of the plant as well as the roots of the plant if the soil is resistant to the introduction of the plant. Traditionally, this may be solved by preparing the ground prior to the transplantation of the plant by e.g. creating a furrow or by creating a depression in the soil where the plant is introduced into a furrow or a depression and where surrounding material is subsequently used to pack the transplanted soil. However, when working in an environment such as at the bottom of a body of water the preparation may be very difficult, which means that other ways have to be utilized to protect the plant.

The casing assembly in accordance with the present disclosure reduces the risk that damage is done to the plant by ensuring that the force application part is located in the vicinity of the second end, and by transferring a force to the second end of the casing it is possible to allow the second end of the casing to enter the soil before any other part of the casing, allowing the second end of the casing to create a depression for the casing in the soil. Thus, the second end is pushed into the soil using the force application part, where the second end pulls the remaining parts of the casing into the soil and thereby ensuring that the force applied to the plant is a pulling force coming from the second end of the casing, i.e. a force that originates below the plant during transplantation and not a force coming from the above, pushing the plant into the soil.

This ensures that the plant is protected during transplantation, that the second end of the casing breaks the ground of the soil, and the side wall of the casing protects the plant during the introduction of the casing assembly into the ground.

In one exemplary embodiment, the force application part may be arranged between a first casing volume and a second casing volume, where a fold on at least one side wall is arranged between the first casing volume and the second casing volume providing a force application part. This means that the casing may hold two plants, and where the first casing volume is on one side of the fold and the second casing volume may be on the opposite side of the fold, where each casing volume is configured to hold a part of a plant.

In one exemplary embodiment, the force application part may have a force application axis, where the force application axis may be parallel to a longitudinal axis of a casing volume, and where the force application axis is arranged at a predetermined distance from the longitudinal axis of the casing volume. The force application axis may be configured to apply a force to the casing, where the connection between the force application part and the second part of the casing transfers the force from a force application axis that is distal to the casing volume axis, to the casing volume axis, and thereby allowing the force to be mechanically transferred from the force application part to the remaining parts of the casing.

In accordance with the present disclosure, the casing assembly may be an anchoring assembly, where the casing assembly may be utilized to anchor a plant in soil. The anchor may be important in relation to the fact that when the plant is transplanted into a soil of a body of water, the plant may have a buoyancy that is positive in the body of water. Thus, if the plant is not anchored in the soil, there is a risk that the plant may be pulled out of the soil due to the buoyancy force of the plant in the body of water.

In one or more exemplary embodiments the force application part may be arranged on an outer surface of the first side wall or the second side wall. The casing volume may be on an inner surface of the first side wall or the second side wall, where the force application part is on an outer surface of the same walls. This means that when a force is applied via the force application part, the plant, which is within the casing volume, will not be directly affected by the force. Thus, when a force application member interacts with the force application part the force application member does not come in direct contact with the plant but only comes in direct contact with an outer surface of the first side wall and/or the second side wall.

In one or more exemplary embodiments the force application part may be in mechanical communication with the second end of the casing, so that a force applied to the force application part in the direction of the longitudinal axis may be transferred to the second end of the casing. The force application part may be in direct or indirect attachment with the second end of the casing, so that when a force is applied to the force application part, the force is transferred to the second end so that at least part of the applied force is transferred from one part of the casing assembly to another part of the casing assembly.

In one or more exemplary embodiments the first end and/or the second end may be closed. By closing the first end and/or the second end it may be possible to protect the plant from the outside of the casing. As an example, when the second end is closed, the casing volume close to the second end is closed to the surroundings of the casing assembly, which means that if the second end is pulled into a soil surface the second end prevents the soil from entering the casing volume. Thus, the soil will slide along the side walls of the casing when the casing enters the soil, and so that when a force is applied to the casing to transplant the plant, the counterforce of the stationary part, i.e. the soil, will not affect the plant directly. However, the counterforce may indirectly affect the plant in that forces pressing on the side walls may squeeze the plant and the casing and thereby ensure that the casing is fixed relative to the soil, when the casing has been introduced into the soil.

In one or more exemplary embodiments the casing assembly may comprise an elongated force application member configured to cooperate with the force application part for applying a force to the force application part. The elongated force application member may be in the form of e.g. an elongated shaft, where the shaft may be actuated manually or automatically, and where one end of the shaft comes into contact with the force application part, and the opposite end of the shaft is utilized to transfer the force necessary to introduce the casing assembly into the soil. The shaft may be a manual shaft, that may be held by a user, or may be part of an automatic transplanter, that may be connected to an actuator, where the actuator is configured to introduce the casing assembly and the plant into the soil.

In one or more exemplary embodiments the first side wall and/or the second side wall may comprise a textile, where the textile has preferably one or more through-going openings. The textile may be configured to hold the plant, and where the outer surface of the casing may function as a friction increasing surface allowing the casing to be held by friction in the soil. The through-going openings may allow the roots to extend through the material and into the soil, so that the plant may take root in the soil without the casing preventing the growth of the roots.

Alternatively, the material of the first side wall and/or the second side wall may be a fibrous material, non-woven material, fabric, or any kind of flexible material. The flexible material may have through-going openings for allowing the roots to penetrate the material.

In one or more exemplary embodiments the first side wall and/or the second side wall may comprise a biodegradable material. The biodegradable material may be configured to dissolve over a predetermined period of time so that when the roots take into the soil the casing will disappear and leave the plant in its transplanted position.

In one or more exemplary embodiments the force application part comprises a force application volume configured to receive an end of a force application member. The force application part may be formed to create a force application volume, where the force application volume may have at least one opening allowing the force application member to enter the force application volume. The force application part may be in the form of a third side wall, which is folded and attached to the second end of the casing so that when a force is applied the force application member is transferred to the second end of the casing. The force application volume may be less than 50% of the size of the casing volume, or may be less than 40% of the size of the casing volume, or may be less than 30% of the size of the casing volume, or may be less than 20% of the size of the casing volume. The force application volume may have a diameter that is equal or larger than the diameter of the force application member.

In one or more exemplary embodiments the force application volume is defined by at least part of the outer surface of the first side wall and/or the second side wall. This means that the casing volume is on one side (inner surface) of the side wall and the force application volume may be on an opposite side (outer surface) of the side wall. Thus, the force application volume may be separated from the casing volume via the side wall, so that the plant is protected from the force application member and the roots of the plant are not damaged by the force application member.

In one or more exemplary embodiments the side wall comprises a flexible sheet and the flexible sheet is folded at the second end to provide a force application volume. The flexible sheet may be folded, where the fold defines the second end of the casing and one part of the flexible sheet on one side of the fold may define the casing volume and another part of the flexible sheet on the opposite side of the fold may define the force application part and/or the force application volume. The transverse sides of the force application part and/or the force application volume may be attached to the transverse sides of the casing volume, and the end of the second part of the flexible sheet is free, creating an opening which extends in a longitudinal direction towards the fold. Thus, the fold may create a bottom of the force application part/volume and where an application of force on the force application part will transmit the force to the second end (on the opposite side of the fold) of the casing, allowing the force application part to be pushed into the soil, and where the pushing force is transformed to a pulling force on the second end of the casing.

In one or more exemplary embodiments the force application part is a part of the second end of the casing. This means that when the casing is transplanted into the soil, a force is applied to the force application part and/or the second end to pull the casing into the soil.

In one or more exemplary embodiments the length of the casing along the longitudinal axis is larger than the width of the casing along the transverse axis. Thus, the casing will have a length that is larger than the width, allowing the casing to have an elongated shape, and where the part of the casing having the transverse length is introduced into the soil. In one or more exemplary embodiments the length of the casing along the longitudinal axis may be equal to or smaller than the width of the casing along the transverse axis. The casing may be formed in a circular shape, polygonal shape or an elliptical shape.

In one or more exemplary embodiments the first side wall and the second side wall are defined by a sheet material, where the sheet material is folded along the second end part or the second transverse part. This means that the casing may be formed by folding a piece of sheet material, where opposing parts of the sheet material that abut each other may be attached to each other to define the casing volume and/or the opening of the casing. Thus, the production of the casing may be done by e.g. folding a sheet material having a predefined size and shape, and where parts of the sheet material are attached to each other to create a casing volume. Furthermore, the force application part may e.g. be formed by folding the casing one more time in a direction different than the first fold, and where the second fold defines the second end of the casing. Thus, the first side wall and the second side wall may be made out of the same flexible sheet material.

In one or more exemplary embodiments the inner surface of the first side wall is attached to the inner surface of the second side wall at the first end part, the second end part, the first transverse part and/or the second transverse part. In one optional embodiment the inner surface of the first side wall is attached to the inner surface of the second side wall which is attached to two or more of the first end part, second end part, first transverse part or second transverse part. The attachments may define a peripheral edge of the casing volume, where the end parts or the transverse parts that are not attached to its opposing side wall may define the opening of the casing volume.

The present disclosure also relates to a method of transplanting a plant in the soil at the bottom of a body of water, where the method comprises the steps of: introducing a root of a plant and/or a seed of a plant into the casing volume of a casing in accordance with the present disclosure, introducing a force application member into the force application part of the casing, introducing the casing and the force application part into the body of water, aligning the second end of the casing with the soil, applying a force to the force application member to force the casing into the soil.

The present disclosure also relates to a transplanting system for transplanting a plant into the soil of a body of water, where the transplanting system comprises: a transplanting apparatus comprising at least one force application part, and a casing assembly in accordance with the present disclosure.

### Brief description of the drawings

The following is an explanation of exemplary embodiments with reference to the drawings, in which;
Fig. 1 shows a top view of a casing in accordance with the present disclosure,
Fig. 2 shows a perspective view of the casing as shown in Fig. 1,
Fig. 3 shows a perspective view of the casing as shown in Fig. 1,
Fig. 4 shows a front view of a casing and a force application member,
Fig. 5 shows a perspective view of a casing having a plant,
Fig. 6 shows a front view of a casing having a plant and a force application member,
Fig. 7 shows a casing being submerged and placed on a soil surface in a body of water, and
Fig. 8 shows a casing and a plant after transplantation of the casing in the soil.

### Detailed description

Various exemplary embodiments and details are described hereinafter with reference to the figures when relevant. It should be noted that the figures may or may not be drawn to scale and that elements of similar structures or functions are represented by reference numerals throughout the figures. It should also be noted that the figures are only intended to facilitate the description of the embodiments. They are not intended as an exhaustive description of the disclosure or as a limitation on the scope of the disclosure. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

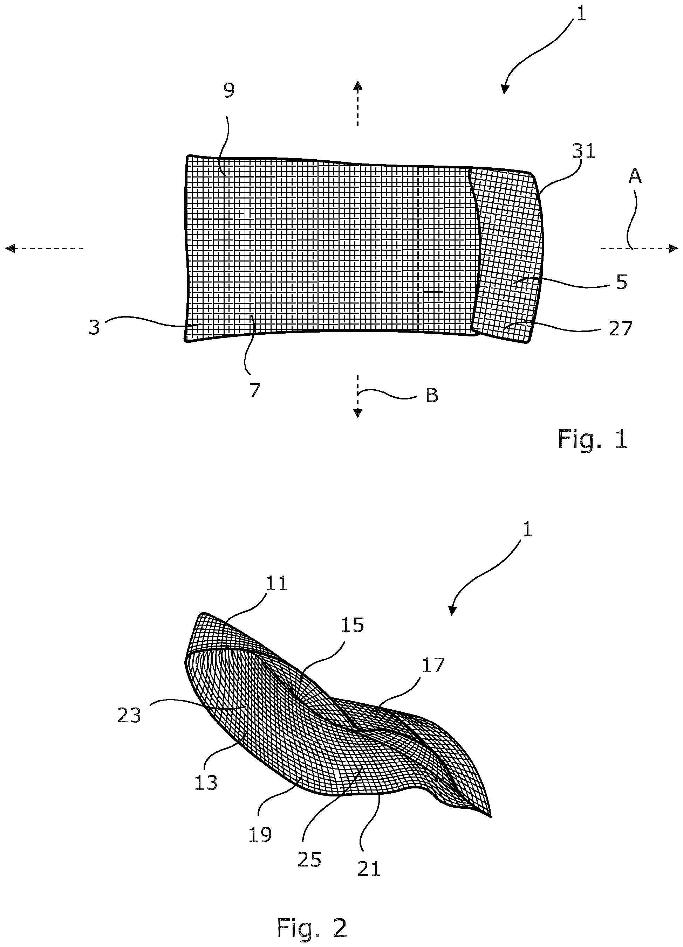

Figs. 1- 4 show a casing 1 for a plant, where the casing has a first end part 3 and a second end part 5, a first peripheral part 7 and a second peripheral part 9, where the casing has a longitudinal axis A extending from the first end part 3 to the second end part 5, and a transverse axis B extending from the first peripheral part 7 to the second peripheral part 9.

The casing 1 may have a first side wall 11 and a second side wall 13 as seen in Fig. 2, where the first side wall 11 has a first inner surface 15 and a first outer surface 17, where the second side wall 13 has a second inner surface 19 and a second outer surface 21. The first side wall 11 and the second side wall may be attached to each other at the first end part 3 and the second end part 5 as well as the first peripheral part 7, where the two side walls 11, 13 define a casing volume 23, and where the casing volume 23 has a casing opening 25 providing access to the casing volume 23. In this embodiment the casing opening 25 is positioned in the second peripheral part 9 of the casing. The opening 25 may be positioned in any part of the casing, where the purpose of the opening 25 is to provide access to the casing volume 23 of the casing 1.

The casing volume 23 may be defined by the first inner surface 15 and the second inner surface 19 of the side walls 11, 13.

The second end part 5 of the casing 1 may have a force application part 27 which is positioned close to the second end 31 of the casing 1, where the force application part 27 is connected to the second end 31 of the casing, so that a force applied in the direction of the longitudinal axis A to in a direction from the first end part 3 to the second end 5, 31 is transferred to the first side wall 11 and or the second side wall 13 via the second end 31, by pulling on the side walls 11, 13 in the direction of the applied force.

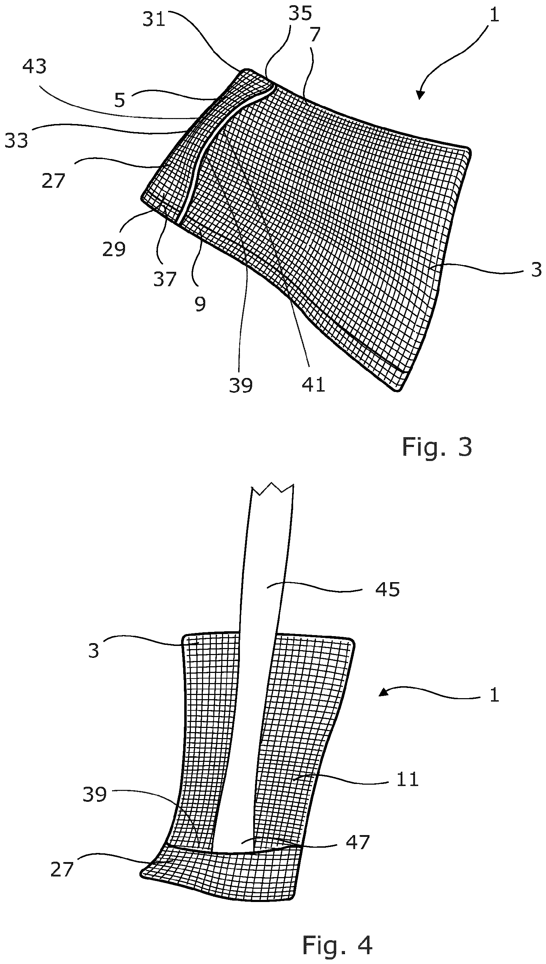

Fig. 3 shows the casing 1, where the force application part 31 is in the form of a folded material 29, where the fold 33 defines the second end 31 of the casing 1, and the first side 35 and the second side 37 of the folded material 29 is attached to the first peripheral part 7 and the second peripheral part 9, and where the force application part 27 defines a force application volume 39 having a force application opening 41 and a closed end 43 defined by the fold 33. This means that the force application part 27 is mechanically coupled to the second end 31 of the casing 1. Furthermore, by providing a force application part 27 which is positioned on the first outer surface 17 or the second outer surface 21, the force application part is positioned on one side of a side wall 11, 13 while the casing volume 23 is on another side of the casing volume 23.

Fig. 4 shows where a force application member 45 having a first end (not shown) and a second end 47 has been positioned inside the force application part 27, so that the second end 47 is positioned inside the force application volume 39. The force application member 45 is separated from the casing volume 21 (shown in Fig. 2) via the first side wall 11, so that the force application member 45 does not come in contact with the casing volume 21.

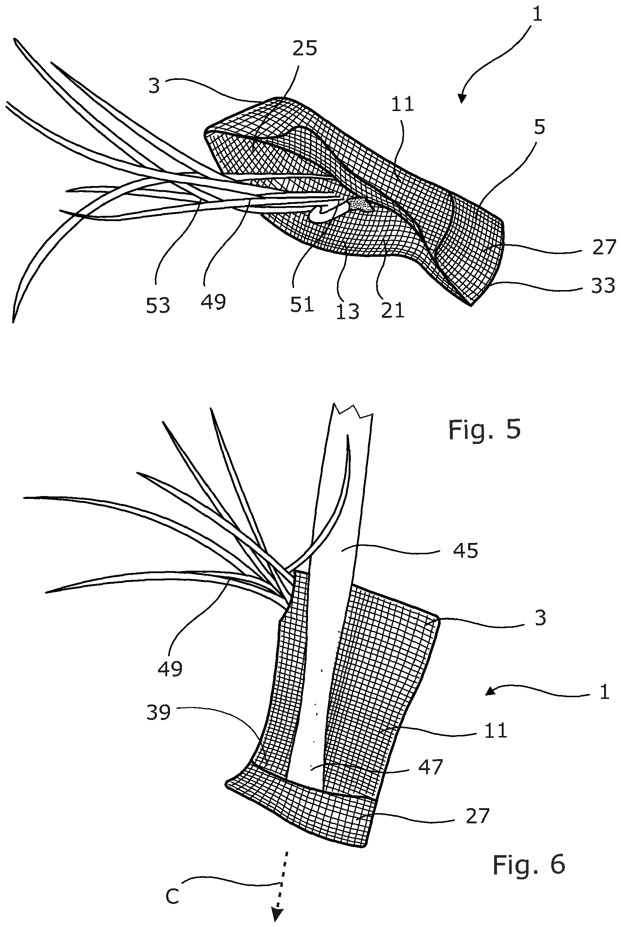

Fig. 5 shows the casing of Fig. 1-4, where a plant 49 has been introduced into the casing volume 21, where the first inner surface 15 and the second inner surface 19 enclose a part of the plant 49. The plant 49 may have a root part 51 and a stem part 53, where a part of the stem part 53 may extend from the casing volume 21 and through the casing opening 25 to the surroundings of the casing 1.

Fig. 6 shows the casing 1 and the plant 49 of Fig. 5, in which a force application member 45 has been inserted inside the force application part 27, so that the force application member may be utilized to push the force application part 27 in the direction of the arrow C, and where the force application part 27 pulls the first side wall 11 and the second side wall 13 in the same direction. This means that the plant 49 inside the casing volume 21 may be pulled in the direction of arrow C.

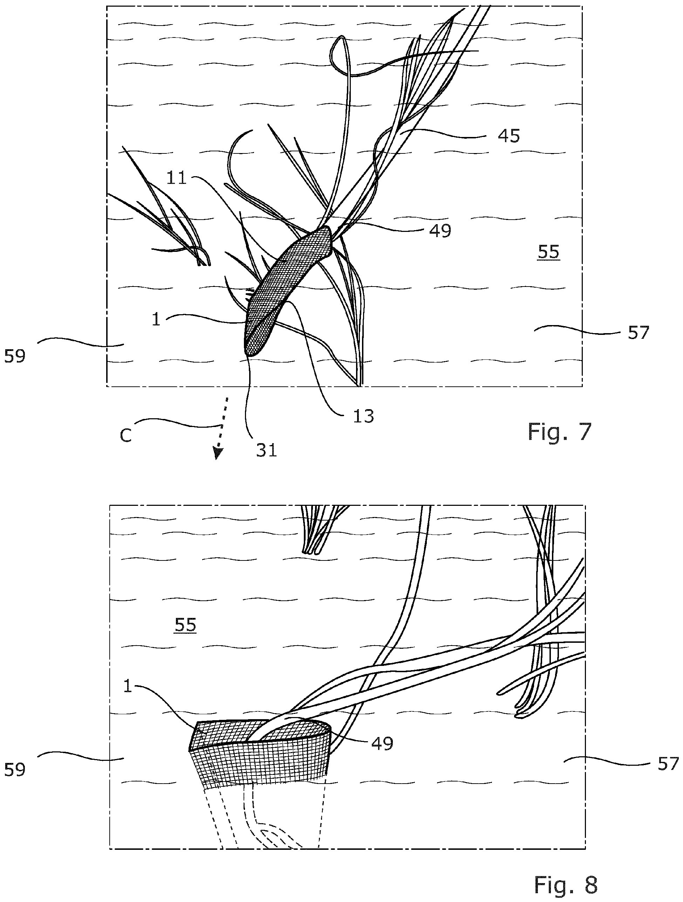

Fig. 7 and Fig. 8 show a casing 1 having a plant 49 similar to that shown in Fig. 5, which is introduced into a body of water 55, where the body of water has a bottom 57, having a soil 59. A force application member 45 has been introduced into the force application part 27 (shown in Fig. 6), where a force in the direction of arrow C may be applied to the force application part 27 to push the force application part 27 and the second end 31 of the casing 1 towards the soil 59. When the second end 31 comes into contact with the soil 59, the force applied to the force application part 27 pulls on the side walls 11, 13 of the casing 1 and thereby pulls the plant 49 into the soil 59 as seen in Fig. 8. When the casing 1 has been introduced into the soil 59, the force application member 45 may be retracted or pulled towards the surface of the body of water 55 and may be utilized to plant the next casing 1 having a plant 49.

When the casing 1 has been introduced into the soil, the soil will apply pressure onto the sides of the casing 1, which may mean that the side walls of the casing are forced in a direction towards each other, and the casing volume may be reduced significantly, so that the casing volume is equal to or only slightly larger than the volume of the plant/seed being held within the casing volume.

The casing 1 may thereby be utilized to protect the plant 49 during transplantation, and where the casing 1 ensures that the force application member 45 does not come into contact with the plant and thereby reducing the risk of damage to the plant 49. Furthermore, as the casing 1 encloses the plant 49, the casing may function as an anchor for the plant 49 until the roots 51 of the plant 49 have taken hold into the soil 59.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering.

Furthermore, the labelling of a first element does not imply the presence of a second element and vice versa.

It is to be noted that the word "comprising" does not necessarily exclude the presence of other elements or steps than those listed.

It is to be noted that the words "a" or "an" preceding an element do not exclude the presence of a plurality of such elements.

It should further be noted that any reference signs do not limit the scope of the claims.

Although features have been shown and described, it will be understood that they are not intended to limit the claimed invention, and it will be made obvious to those skilled in the art that various changes and modifications may be made without departing from the spirit and scope of the claimed invention. The specification and drawings are, accordingly, to be regarded in an illustrative rather than restrictive sense. The claimed invention is intended to cover all alternatives, modifications, and equivalents.

### List of references

- 1: Casing
- 3: First end part
- 5: Second end part
- 7: First peripheral part
- 9: Second peripheral part
- 11: First side wall
- 13: Second side wall
- 15: First inner surface
- 17: First outer surface
- 19: Second inner surface
- 21: Second outer surface
- 23: Casing volume
- 25: Casing opening
- 27: Force application part
- 29: Folded material
- 31: Second end of casing
- 33: Fold
- 35: First side of fold
- 37: Second side of fold
- 39: Force application volume
- 41: Force application opening
- 43: Closed end of force application part
- 45: Force application member
- 47: Second end of force application member
- 49: Plant
- 51: Root of plant
- 53: Stem of plant
- 55: Body of water
- 57: Bottom of water
- 59: Soil
- A: Longitudinal axis
- B: Transverse axis
- C: Direction of transplantation

## Claims

1. A casing assembly for a root of a plant and/or a seed of a plant configured to protect at least a part of the plant during transplantation of the plant, where the casing assembly comprises:
- at least one casing defining a casing volume for holding a part of a plant, where the casing comprises a first end part, a second end part, a first peripheral part and a second peripheral part, where the casing has a longitudinal axis extending from the first end part to the second end part, and a transverse axis extending from the first peripheral part to the second peripheral part,
- wherein the casing comprises a first side wall and a second side wall having a first inner surface and a second inner surface, respectively, defining the casing volume and a first outer surface and a second outer surface, respectively,
- wherein the casing volume has an opening, the opening is positioned in the first end part and/or the first peripheral part and/or in a side wall of the casing,
- where the second end part comprises a force application part, so that when the casing is transplanted into a soil material the casing is pulled into the soil via the second end.

2. A casing assembly in accordance with claim 1, wherein the force application part is arranged on an outer surface of the first side wall or the second side wall.

3. A casing assembly in accordance with any one of the preceding claims, wherein the force application part has a force application axis, where the force application axis is parallel to a longitudinal axis of a casing volume, and where the force application axis is arranged at a predetermined distance from the longitudinal axis of the casing volume.

4. A casing assembly in accordance with any one of the preceding claims, wherein the force application part is in mechanical communication the second end of the casing, so that a force applied to the force application part in the direction of the longitudinal axis is transferred to the second end of the casing.

5. A casing assembly in accordance with any one of the preceding claims, wherein the first end and/or the second end are closed.

6. A casing assembly in accordance with any one of the preceding claims, wherein the casing assembly comprises an elongated force application member, configured to cooperate with the force application part for applying a force to the force application part.

7. A casing assembly in accordance with any one of the preceding claims, wherein the first side wall and/or the second side wall comprises a textile, where the textile has preferably one or more through-going openings.

8. A casing assembly in accordance with any one of the preceding claims, wherein the force application part comprises a force application volume configured to receive an end of a force application member.

9. A casing assembly in accordance with claim 8, where the force application volume is defined by at least part of the outer surface of the first side wall and/or the second side wall. Thus, the casing volume is on one side of the side wall and the force application volume is on an opposite side of the side wall.

10. A casing assembly in accordance with any one of the preceding claims, wherein the side wall comprises a flexible sheet and where the flexible sheet is folded at the second end to provide a force application volume.

11. A casing assembly in accordance with any one of the preceding claims, wherein the first side wall and the second side wall are defined by a sheet material, where the sheet material is folded along the second end part or the second transverse part.

12. A casing assembly in accordance with any one of the preceding claims, wherein the inner surface of the first side wall is attached to the inner surface of the second side wall at the first end part, the second end part, the first transverse part and/or the second transverse part.

13. A method of transplanting a plant in the soil at the bottom of a body of water, where the method comprises the steps of:
- introducing a root of a plant or a seed of a plant into the casing volume of a casing in accordance with any of the claims 1-12,
- introducing a force application member into the force application part of the casing,
- introducing the casing and the force application part into the body of water,
- aligning the second end of the casing with the soil,
- applying a force to the force application member to force the casing into the soil.

14. A transplanting system for transplanting a plant into the soil of a body of water, where the transplanting system comprises:
- a transplanting apparatus comprising at least one force application part, and
- a casing assembly in accordance with any of the claims 1 - 13.
